# EUROPEAN PATENT APPLICATION

(11) **EP 1 048 279 A2**
(43) Date of publication of application: **02.11.2000**
(21) Application number: 00108474.8
(22) Date of filing: 18.04.2000
(51) Int. Cl.: A61F 13/56, A61F 13/514

(54) **Breathable disposable absorbent articles**

(30) Priority: 30.04.1999 US 302335
(71) Applicant: First Quality Enterprises, Inc., McElhattan, Pennsylvania 17748 (US)
(72) Inventor: Karami, Hamzeh, McElhattan, PA 17748 (US)
(74) Representative: Motsch, Andreas

(57) **Abstract**

A breathable disposable absorbent article such as, for example, a diaper, is provided which comprises a top sheet, a backsheet and an absorbent layer therebetween. The top sheet and the backsheet are sealed together to form a unitary structure and include a medial cutout portion on each side which define openings for the leg of the wearer. One or more so-called landing zones are provided on the backsheet, adapted to releasably adhere to fastening tabs located at each side of the article. A liquid impervious sheet or film is laminated to the top surface of the backsheet in order to prevent leakage of fluids and exudates.

## Description

### FIELD OF THE INVENTION

This invention relates to disposable absorbent articles such as diapers, briefs, pants, incontinent garments and the like, and it is particularly related to such articles which exhibit breathability, and are therefore commonly referred to in the art as "breathable" absorbent articles.

### BACKGROUND OF THE INVENTION

Disposable absorbent articles such as disposable baby diapers and adult incontinent briefs, underpants, guards and the like articles are widely used in the homes and in various health care facilities and institutions. Indeed the use of such articles has become a sanitary practice, and while initially such absorbent articles were used mostly for baby care, more recently their use has been expanded for adults as well. In both instances, the absorbent article must be designed to effectively prevent leakage of urine and other fecal materials, while insuring body fit and comfort.

Present commercially available absorbent articles are generally unitary in structure, pre-shaped and pre-folded and comprise an absorptive pad having a liquid permeable top sheet facing the wearer's body, a liquid impermeable backsheet on the opposite side, and an absorbent sheet or panel disposed between the top sheet and the backsheet. The absorbent article comprises a front side portion, a crotch portion and a backside portion, and further includes elastic members along the circumference of the waist and around the leg openings. The underlying objective in the design and construction of these absorbent articles has been to maximize prevention of leakage of body fluids and fecal materials through the legs' openings. The prior art workers in this field have recognized that while the backsheet of the absorbent article must be liquid impermeable, they have also recognized that, in addition, the backsheet must be breathable in order to afford comfort to the wearer. Indeed there are numerous patents which disclose diapers and incontinent adult briefs having breathable backsheets. For example, United States Patent No. 5,263,948 issued to Karami at al. on November 23, 1993 describes breathable disposable diapers and contains a list of representative patents which illustrate the state of the art of breathable absorbent articles. The aforementioned Karami et al. patent describes a breathable absorbent article aimed at providing optimum breathability while preventing fluid and solid leakages through the side edges of the crotch area of the diaper.

A breathable disposable diaper is also described in United States Patent No. 4,900.137, issued to Kenneth B. Buell on February 13, 1990. In the Buell patent, the diaper is provided with breathable leg cuffs formed of a materiel which permits passage of the vapors while retarding fluid leakage.

An absorbent article with breathable side panels is described in United States Patent No. 5,628,737 issued to Dobrin et al. on May 13, 1997.

Notwithstanding attempts by others in this industry to provide absorbent articles which prevent leakage of urine and other body exudates, and which also exhibit satisfactory breathability and comfort to the wearer, there is still need for providing such absorbent articles which exhibit optimum improvements in these characteristics.

Accordingly, it is a general object of the present invention to provide breathable absorbent articles such as baby diapers, adult incontinent underpants, brief, guards and the like.

It is another object of the present invention to provide such absorbent articles which, due to their unique construction provides optimum breathability while affording excellent body fit and comfort to the wearer.

It is a further object of this invention to provide absorbent articles of the type herein described which are relatively simple to manufacture at affordable commercial prices.

The foregoing and other objects and features of the present invention will be more fully comprehended and appreciated from the ensuing detailed description and the accompanying drawing which form parts of this application.

It must be understood throughout this application that the term "breathable" refers to articles and garments having a backsheet which is either totally, or partially breathable, both of which hereinafter will be referred to as "breathables".

### BRIEF DESCRIPTION OF THE DRAWINGS

In the various figures which constitute the drawings illustrating the present invention, and wherein like reference numerals refer to like parts:
Figure 1 is a plan stretched view of the back of a diaper made according to this invention;
Figure 2 is of a sectional view taken along the line 2-2 of Figure 1;
Figure 3 is a sectional view similar to Figure 2 but taken along the line 3-3 of Figure 1; and
Figure 4 is an elevational sectional view taken along the line 4-4 of Figure 1.

### SUMMARY OF THE INVENTION

The objects of this invention are achieved by providing a breathable disposable absorbent article which comprises:
(a) a backsheet having opposed side edges and opposed front waist and back edges connecting said side edges, and having a medial cutout portion on each of said side edges,
(b) a top sheet having corresponding opposed side edges and corresponding opposed front waist and back edges connecting said side edges, and a medial cutout portion on each of said side edges, said backsheet and said topsheet having the same general configuration, and being sealed together at their respective edges so as to form a unitary structure having leg openings defined by said medial cutout portions,
(c) an absorbent layer disposed between said topsheet and said backsheet for receiving body fluids and exudates,
(d) one or more releasable fastening tabs at each of said side edges disposed at one end of said absorbent article, and
(e) one or more landing zones, having an inner adherent surface adherent to said backsheet, and an opposed outer surface adapted to be releasably engaged to the fastening tabs.

These landing zones may be perforated and their outer surfaces may be treated with silicone, wax or some other suitable material for releasably adhering to the fastening tabs. A liquid and vapor impervious layer such as, e.g., a polyethylene, is interposed between the absorbent layer and the backsheet, and is preferably adhered or laminated to the backsheet to prevent leakage of the body fluid and exudates.

### DETAILED DESCRIPTION OF THE INVENTION

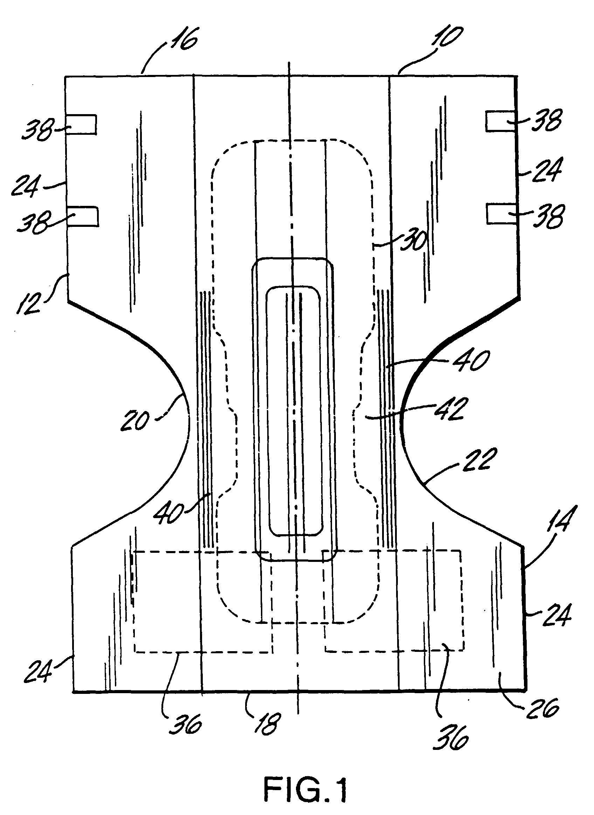

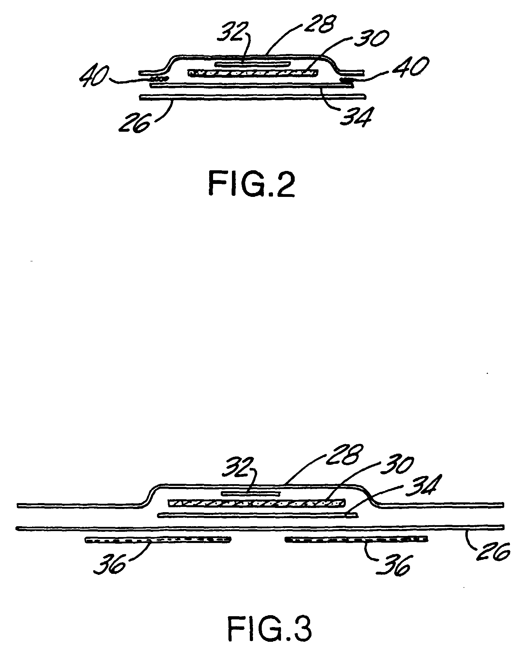

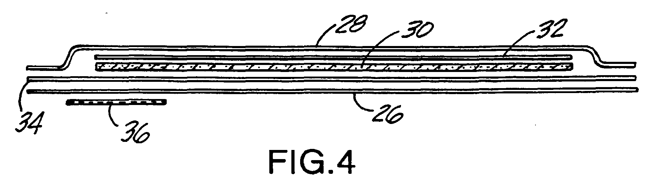

Referring to Figure 1, there is shown a diaper generally designated as 10 which is defined by the opposed side edges 12 and 14 and the end edge (back waist) 16 and end edge (front waist) 18 which connect the side edges. Each of the side edges 12 and 14 has a medial cutout portion which defines the leg openings 20 and 22 for insertion of the legs of the wearer there through when the end edge 18 (front waist end) is folded onto the end edge 16 (back waist end) to form the diaper structure as it is worn by the user. As is further shown in Figure 1, each of the side edges 12 and 14 has laterally extending side ears or panels 24. The diaper 10 also comprises a breathable backsheet 26, a fluid pervious front or top sheet 28 facing the body of the wearer and an absorbent pad or layer 30 disposed between the top sheet and the back sheet as shown in Figures 2-4. The top sheet is generally coextensive with the backsheet although this is not strictly necessary. Also shown in Figures 2-4, is an acquisition layer 32 interposed between the top sheet 28 and the absorbent pad 30. The acquisition layer serves to temporarily retain the body exudates and slowly distribute them through the absorbent pad 30 in order keep the skin dry. Referring again to Figures 2-4, the composite layers also include the layer 34, generally made of liquid and vapor impermeable polyethylene, spanning the length of the absorbent article and extending laterally toward the sides of the diaper, ending at the cutout portions 20, 22. The polyethylene film 34 may be laminated to the backsheet 26 as seen from Figures 2-4 and thus provides an area which is liquid and vapor impervious.

As seen from Figure 1, the backsheet 26 is provided with a pair of opposed, perforated (or non-perforated) films or landing zones 36, usually made of flexible polyolefin plastic such as polyethylene or polypropylene, a polyester, a cellulose ester or some other plastic material known in the art, but preferably it is polyethylene or polypropylene. A pair of releasable tabs, e.g., adhesive tabs 38 are provided at the side panels or ears 24 on the front waist side edge 16 of the absorbent article such that when the back waist end 18 of the diaper 10 is folded onto the front waist end 16 of the diaper, the tabs 38 are securely adhered onto the corresponding surfaces of the landing zones 36. Thus, the landing zone 36 has an inside surface which is adherent to the backsheet 26, and an exterior surface which is adapted to be releasably fastened to the tabs 38. To this end, the exterior surface of the landing zone 36 may be treated with silicone or other suitable material so as to render this surface releasably adherent to the tabs 38. Instead of using a release agent or in cooperation therewith, the exterior surfaces of the landing zones may be embossed in order to reduce the adhesion forces between the fastening tabs and the exterior surfaces of these zones. It can be seen, therefore, that in use, when the back waist 18 is folded onto the front waist 16 the releasable tabs 38 can be fastened at any point on the surfaces of the landing zones 36 thus affording adjustment and improved fit of the diaper around the waist of the wearer. Although two perforated landing zones are shown in Figure 1, one or more such zones may be provided which may or may not be perforated, as desired.

The diaper 10 also comprises at least one or more elastic members or bands 40 disposed on the opposite sides 12 and 14. Preferably, 2 to 4 such elastic bands are used and act as "gatherers" in the crotch region 42. These elastic members are sometimes referred to in this art as crotch elastics and serve to prevent fluid leakage from the crotch area or leg openings of the diaper. The elastic bands 40 are usually under tension and are adhesively secured at the sides of the article between the top or cover sheet 28 and the polyethylene backing film 34.

In making the absorbent article of the present invention, the top or cover sheet 28 is made of spunbond nonwoven polypropylene which is available from First Quality Fibers, Inc., McElhattan, PA. The acquisition layer 30 is usually made of chemically bonded nonwoven polypropylene available from American Nonwovens, Columbus, Missouri. Preferably the width of the acquisition layer 30 is substantially the same as the width of the absorbent pad 30. This pad may be made of wood pulp fibers and superabsorbent polymers such as IM 7000 series available from Clariant Products, Inc., Portsmouth, VA, and Chemdal 200 Series, available from Chemdal, Inc., Palantin, III. Alternatively, the absorbent pad 30 may be of dual construction, in which case the absorbent polymer may be securely positioned between each layer of the absorbent material. As seen in Figure 1, the absorbent pad 30 does not extend to the side panels or ears 24 and, therefore, in the construction contemplated by the present invention, the absorbent pad is on the target area where it can afford maximum protection against fluid leakage.

The layer 34 is a polyethylene backing film and is generally non-porous and is liquid, air and preferably vapor impermeable. This layer is placed under this absorbent layer 30 in order to prevent the body exudates from leaking and thus soiling the user's bed and clothing. This film usually covers the full area under the absorbent pad 30. In a preferred construction, the backing film 34 is adhesively or thermally laminated to the backsheet 26, which itself is made of spunbond-melt bond spunbond (SMS) nonwoven polypropylene available from First Quality Fibers, McElhattan, Pennsylvania. Polyethylene suitable as backing film for making the diapers of this invention are available from Clopay Plastics, Cincinnati, Ohio. Alternatively, a layer of hydrophobic tissue may be used instead of, or in conjunction with the polyethylene layer. The hydrophobic tissue may be made of cellubasic fibers bonded together thermally, chemically or by the use of suitable sizing agents. The hydrophobic tissue layer is microporous, i.e., comprises extremely small pores which permit passage of air and moisture vapor, but prevents, or is resistant to, passage of liquid. For improved effectiveness, the hydrophobic tissue may be laminated (cold or hot) to the nonwoven backsheet.

One special feature of the absorbent article of this invention is that it affords optimum breathability to the wearer. In one embodiment, the side panels or ears 24 may be porous in order to permit passage of air therethrough. The landing zone 36 may be perforated and thus not only serve to impart breathability to the absorbent article but also provide for zones of engagement with the fastening tabs 34 when the back of the diaper is folded onto the front of the diaper for use by the wearer.

While the present invention has heretofore been described with certain degrees of particularities and with reference to absorbent diapers and the like garments, it must be understood that several modifications may be made in the structure of the article which are apparent from the forgoing disclosure. Such modifications include the selection of specific materials used to fabricate the different layers, the degree of porosity, size, configuration and distribution of the pores when using porous landing zones. Such modifications are nevertheless within the scope and contemplation of this invention.

## Claims

1. A breathable disposable absorbent article comprising:
(a) a backsheet having opposed side edges and opposed front waist and back waist edges connecting said side edges, each of said side edges having a medial cutout portion,
(b) a top sheet having opposed side edges and corresponding opposed front waist and back waist edges connecting said side edges, each of said side edges having a medial cutout portion, said backsheet and said topsheet being sealed together at their respective edges so as to form a unitary structure having leg openings defined by said medial cutout portions,
(c) an absorbent layer disposed between said top sheet and said backsheet for receiving body fluids and exudates,
(d) at least one releasable fastening means at each of said side edges disposed at one end of said absorbent article, and
(e) at least one zone disposed on said backsheet, said zone having an inside surface adherent to said backsheet, and an opposed outer surface adapted to be releasably engaged to said fastening means.

2. A breathable disposable absorbent article as in claim 1 having a pair of spaced apart landing zones, each landing zone having an inside surface adherent to said backsheet, and an opposed outer surface adapted to be releasably engaged to said fastening means.

3. A breathable disposable absorbent article as in claim 1 or 2 wherein said backsheet is a composite of two layers, a nonwoven layer and at least one other layer selected from the group consisting of polyethylene and polypropylene.

4. A breathable disposable absorbent article as in any of the claims 1 to 3 wherein said backsheet is a composite of, from top to bottom, a film made of polypropylene, polyethylene or mixtures thereof, spunbond-meltblown-spunbond nonwoven, and a film made of spunbond meltblown nonwoven, respectively.

5. A breathable disposable absorbent article as in any of the claims 1 to 4 further including a layer disposed between said backsheet and said absorbent layer, wherein said layer is made of a material selected from the group consisting of polyethylene and hydrophobic tissue.

6. A breathable disposable absorbent article as in any of the claims 1 to 5 wherein said zone is a perforated zone.

7. A breathable disposable absorbent article comprising:
(a) a backsheet having opposed side edges and opposed waist and back edges connecting said side edges, each of said side edges having a medial cutout portion,
(b) a top sheet having opposed side edges and opposed front waist and back waist edges connecting said side edges, each of said side edges having a medial cutout portion,
said backsheet and said topsheet being sealed together at their respective edges so as to form a unitary structure having leg openings defined by said medial cutout portion,
(c) an absorbent layer disposed between said top sheet and said backsheet for receiving body fluids and exudates,
(d) a liquid impervious layer attached to said absorbent layer between said absorbent layer and said backsheet,
(e) at least one releasable fastening means at each of said side edges disposed at one end of said absorbent article, and
(f) at least one zone disposed on said backsheet, said zone having an inside surface adherent to said backsheet, and an opposed outer surface adapted to be releasably engaged to said fastening means.

8. A breathable disposable absorbent article as in claim 7 where said zone is a perforated zone.

9. A breathable disposable absorbent article as in claim 8 wherein two spaced apart zones are provided on said backsheet.

10. A breathable absorbent article as in claim 9 wherein at least one of said zones is a perforated zone.
